(19) Europäisches Patentamt
European Patent Office
Office européen des brevets

(11) Publication number: **0 085 407 A1**

(12) **EUROPEAN PATENT APPLICATION**

(21) Application number: 83100772.9

(22) Date of filing: 27.01.83

(51) Int. Cl.³: C 07 D 487/04
//A61K31/40, (C07D487/04, 209/00, 205/00)

(30) Priority: 28.01.82 JP 11010/82
13.03.82 JP 38869/82

(43) Date of publication of application:
10.08.83 Bulletin 83/32

(84) Designated Contracting States:
AT BE CH DE FR GB IT LI NL

(71) Applicant: SANRAKU-OCEAN CO., LTD.
15-1, Kyobashi 1-chome Chuo-ku
Tokyo(JP)

(72) Inventor: Yoshioka, Takeo
Green Haitsu 3-3103 1959 Kamitsuchidana
Ayase-shi Kanagawa-ken(JP)

(72) Inventor: Kojima, Ikuo
23-1 Nagatakita 2-chome Minami-ku
Yokohama-shi Kanagawa-ken(JP)

(72) Inventor: Okabe, Mitsuyasu
Fujisawa Seibu Danchi 1-1-1074 3874 Ohba
Fuisawa-shi Kanagawa-ken(JP)

(72) Inventor: Shimauchi, Yasutaka
1-101-13, Yurigaoka Ninomiya-machi
Naka-gun Kanagawa-ken(JP)

(72) Inventor: Fukagawa, Yasuo
1194-146 Imaizumi
Kamakura-shi Kanagawa-ken(JP)

(72) Inventor: Ishikura, Tomoyuki
22-32, Kowada 1-chome
Chigasaki-shi Kanagawa-ken(JP)

(72) Inventor: Watanabe, Azuma
Sanraku Hodogayaryo Nai 2-23-2 Nagatakita
Minami-ku Yokohama-shi Kanagawa-ken(JP)

(74) Representative: Patentanwälte Müller-Boré, Deufel, Schön, Hertel, Lewald, Otto
Postfach 86 07 20 Siebertstrasse 4
D-8000 München 86(DE)

(54) **Derivatives of antibiotics 0A-6129A, 0A-6129B1 and 0A-6129B2 and their preparation method.**

(57) Novel derivatives of antibiotics OA-6129A, OA-6129$B_1$ and OA-6129$B_2$ having the formula:

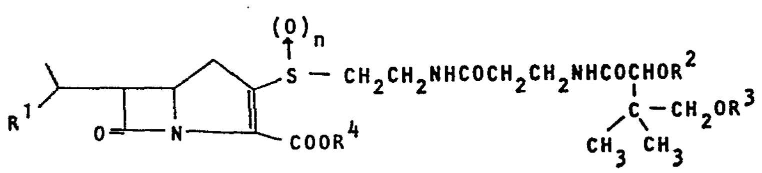
(1)

wherein
$R^1$ represents a hydrogen atom or a hydroxyl group,
n represents zero or 1,
$R^2$ and $R^3$ each represent a hydrogen atom or together with each other a ketal or acetal group, and
$R^4$ represents a substituted or unsubstituted benzyl group,
and processes for producing them by allowing the antibiotics mentioned above to react to form ketal or acetal, or S-oxides thereof.

The present derivatives show antimicrobial activity and are useful as intermediates for production of valuable carbapenem antibiotics.

EP 0 085 407 A1

SUMMARY OF THE INVENTION

The present invention relates to derivatives of antibiotics OA-6129A, OA-6129$B_1$ and OA-6129$B_2$, and more specifically to novel compounds having the following formula:

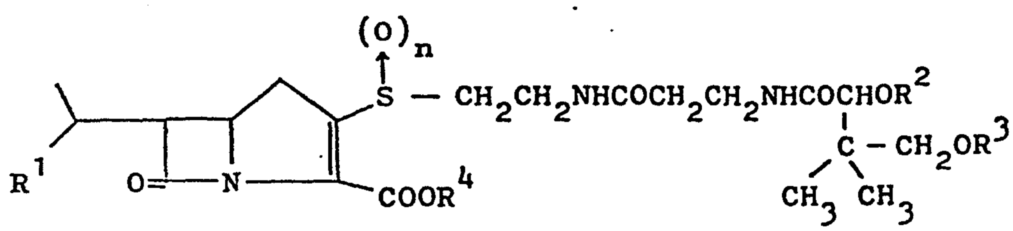

(1)

wherein

n represents zero or 1,

$R^1$ represents a hydrogen atom or a hydroxyl group,

$R^2$ and $R^3$ represent together with each other a group having the formula:

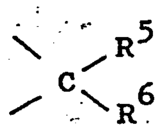

when n is zero,

wherein

$R^5$ and $R^6$ are same or different each other and each represent a hydrogen atom, lower alkyl, phenyl group, a group having a carbon ring of 5 to 6 carbon atoms or diphenylmethyl group, or

$R^2$ and $R^3$ each represent a hydrogen atom or together with each other a group having the formula:

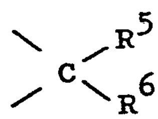

when n is 1,

wherein

$R^5$ and $R^6$ are as mentioned above, and

$R^4$ represents a substituted or unsubstituted benzyl group,

and to their preparation method.

BACKGROUND OF THE INVENTION

Antibiotics containing 7-oxo-1-azabicyclo [3.2.0] hept-2-ene-2-carboxylic acid ring represented by the following formula:

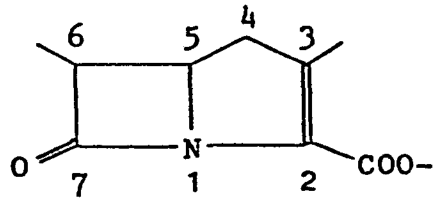

have generally been known to show high antimicrobial and β-lactamase -inhibitory activities. Various kinds of 7-oxo-1-azabicyclo [3.2.0] hept-2-ene-2-carboxylic acid derivatives have been prepared by fermentation, semi-synthetic or synthetic processes.

Some examples are as follows: thienamycin (J. Antibiotics 32, 1 - 12 (1979)), epithienamycins (Abstracts Nos. 80 and 81, 17th Interscience Conference on Antimicrobial Agents and Chemotherapy), N-acetyl-thienamycin (German Patent 2652681, 1977), olivanates (J. Antibiotics 32, 262 - 286 (1979)), PS-6 and PS-7 (E.P.C.Patent 1567) and a process for preparing novel

carbapenem antibiotics by substituting the side chain at 3-position of PS-5 or PS-6 (EUROPEAN PATENT APPLICATION Publication Number 11172).

DETAILED DESCRIPTION OF THE INVENTION

The present invention relates to derivatives of antibiotics OA-6129A, OA-6129$B_1$ and OA-6129$B_2$, and more specifically to novel compounds having the following formula:

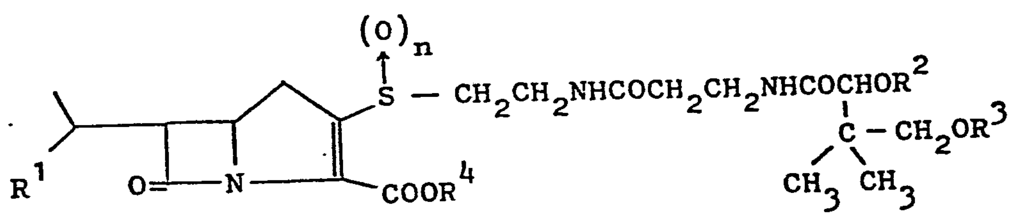

(1)

wherein

n represents zero or 1,

$R^1$ represents a hydrogen atom or a hydroxyl group,

$R^2$ and $R^3$ represent together with each other a group having the formula:

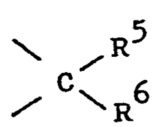

when n is zero,

wherein

$R^5$ and $R^6$ are same or different each other and each represent a hydrogen atom, lower alkyl, phenyl group, a group having a carbon ring of 5 to 6 carbon atoms or diphenylmethyl group, or

$R^2$ and $R^3$ each represent a hydrogen atom or together with each other a group having the formula:

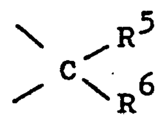

when n is 1,

wherein

$R^5$ and $R^6$ are as mentioned above, and

$R^4$ represents a substituted or unsubstituted benzyl group,

and to their preparation method.

Compounds according to the present invention having the formula mentioned above are obtained from a compound selected as a starting material from a group consisting of antibiotics OA-6129A having the formula:

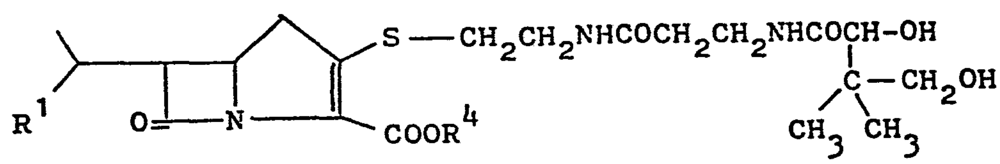

(1-a)

wherein

$R^1$ represents a hydrogen atom,

antibiotic OA-6129$B_1$ having the formula as mentioned above, wherein

$R^1$ represents a hydroxyl group and 5,6-positions form cis-stereostucture,

and antibiotic OA-6129$B_2$ having the formula as mentioned above,

wherein

$R^1$ represents a hydroxyl group and 5,6-positions form trans-stereostructure,

which are structually characterized by the presence of a pantetheinyl group at 3-position and an ethyl or 1-hydroxyethyl group at 6-position of 7-oxo-1-azabicyclo [3.2.0] hept-2-ene-2-carboxylic acid already applied for patent by the present inventors (EUROPEAN PATENT

APPLICATION Publication Number 48999). They are novel cyclic acetal or ketal derivatives, or their S-oxides, the hydroxyl of pantoyl group thereof forming acetal or ketal.

The derivatives according to the present invention are useful as antimicrobial agents and intermediates for synthesis of antimicrobial agents.

Antibiotics having formula 1-a are specifically more stable than known compounds that have the same basic structure. The compounds of the present invention and salts thereof have high antimicrobial and β-lactamase-inhibitory activities, and also an ability to stimulate synnergistically the antimicrobial activity of penicillin- and cephalosporin-antibiotics on β-lactamase-producing microorganisms. Therefore they are useful as antimicrobial agents . In addition the compounds of the present invention are useful as intermediates for production of novel or known valuable derivatives, because, among the compounds of the present invention, the derivatives having the formula:

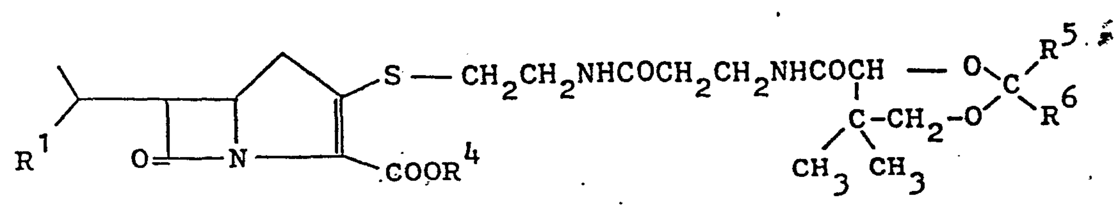

(1-b)

wherein

$R^1$, $R^4$, $R^5$ and $R^6$ are as mentioned above,

retaining the specific properties of compounds having formula 1-a, show improved solubility to organic solvents and can be easily subjected to various reactions in organic syntheses, and also the derivatives having formula 1-b,

wherein

$R^1$ represents a hydroxyl group,

can be selectively protected only at the hydroxyl of pantoyl group.

Derivatives having the formula:

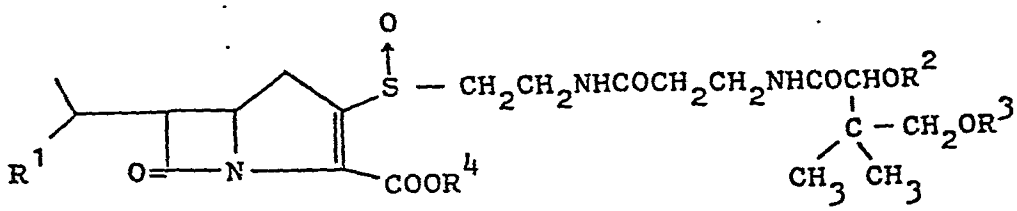

(1-c)

wherein

$R^1$, $R^2$ and $R^3$ are as mentioned above, and

$R^4$ represents a substituted or unsubstituted benzyl group,

can be used in a process for producing derivatives

substituted at their 3'-side chain having the following formula:

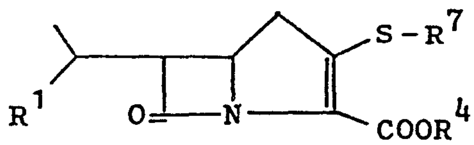

wherein

$R^1$ and $R^4$ are as mentioned above and

$R^7$ is as mentioned below,

by allowing the compound of formula 1-c to react with a thiol compound having the formula:

$R^7$ - SH

wherein

$R^7$ represents a hydrogen atom or a monovalent organic group,

or reactive derivatives thereof, which is included in the invention completed according to the process already disclosed by the present inventors in EUROPEAN PATENT APPLICATION Publication Number 11173 mentioned above. That is, they are useful as intermediates to synthesize derivatives substituted at 3-side chain of carbapenem antibiotics.

Substituents on the benzene ring of substituted

benzyl group used in the present invention are, for example, lower alkyl group such as methyl and ethyl, lower alkoxy group such as methoxy and ethoxy, halogen atom such as chlorine and fluorine , and nitro group. Such a substituted benzyl group is, for example, p-nitrobenzyl, p-bromobenzyl, p-methylbenzyl, 2,4-dinitrobenzyl, and p-methoxybenzyl group.

The lower alkyl group is a group of 1 to 7 carbon atoms, preferably 1 to 4 carbon atoms, for example, such as methyl, ethyl, n-propyl, isopropyl, n-butyl and isobutyl group. As the group having a carbon ring of 5 to 6 carbon atoms, cyclopentane and cyclohexane are mentioned. Therefore, as examples of the group having the formula:

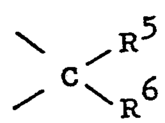

in the case of a combination of $R^5$ and $R^6$, hydrogen atom/hydrogen atom, methyl/methyl, hydrogen atom/methyl, hydrogen atom/phenyl, hydrogen atom/ethyl, methyl/phenyl, phenyl/phenyl, benzyl/benzyl or methyl/benzhydryl, and in the case of a group formed from $R^5$ together with $R^6$, cyclohexylidene group, are mentioned.

As examples of the compounds provided by the present invention, 6-ethyl-3-isopropylidene pantetheinyl-7-oxo-1-azabicyclo [3.2.0] hept-2-ene-2-carboxylic acid p-nitrobenzyl ester, 6-(1-hydroxyethyl)-3-isopropylidenepatetheinyl-7-oxo-1-azabicyclo [3.2.0] hept-2-ene-2-carboxylic acid p-nitrobenzyl ester, 3-cyclohexylidenepantetheinyl -6-ethyl-7-oxo-1-azabicyclo [3.2.0] hept-2-ene-2-carboxylic acid p-nitrobenzyl ester, 3-cyclohexylidenepantetheinyl-6-(1-hydroxyethyl)-7-oxo-1-azabicyclo [3.2.0] hept-2-ene-2-carboxylic acid p-benzyl ester, 6-ethyl-3-methylidenepantetheinyl-7-oxo-1-azabicyclo [3.2.0] hept-2-ene-2-carboxylic acid benzyl ester, 6-(1-hydroxyethyl)-3-methylidene-pantetheinyl-7-oxo-1-azabicyclo [3.2.0] hept-2-ene-2-carboxylic acid p-nitrobenzyl ester, 3-ethylidene pantetheinyl-6-(1-hydroxyethyl)-7-oxo-1-azabicyclo [3.2.0] hept-2-ene-2-carboxylic acid o,p-dinitrobenzyl ester, 3-benzylidenepantetheinyl-6-ethyl-7-oxo-1-azabicyclo [3.2.0] hept-2-ene-2-carboxylic acid o,p-dinitrobenzyl ester, 3-benzylidenepantetheinyl-6-(1-hydroxyethyl)-7-oxo-1-azabicyclo [3.2.0] hept-2-ene-2-carboxylic acid o,p-dinitrobenzyl ester, 6-(1-hydroxyethyl)-3-(1-methylbenzylidene)-pantetheinyl-7-oxo-1-azabicyclo [3.2.0] hept-2-ene-2-carboxylic acid p-nitrobenzyl ester, 3-dibenzylidene-pantetheinyl-6-(1-hydroxyethyl)-7-oxo-1-azabicyclo [3.2.0] hept-2-ene-2-carboxylic acid benzyl ester, 6-ethyl-3-ethylidenepantetheinyl-7-oxo-1-azabicyclo [3.2.0] hept-2-ene-2-carboxylic acid p-nitrobenzyl ester, 3-ethylidenepantetheinyl-6-(1-hydroxyethyl) -7-oxo-1-azabicyclo [3.2.0] hept-2-ene-2-carboxylic acid p-nitrobenzyl ester, 6-ethyl-3-pantetheinyl-7-oxo-1-azabicyclo [3.2.0] hept-2-ene-2-carboxylic acid p-nitrobenzyl ester sulfoxide, 6-ethyl-3-isopropylidenepantetheinyl-7-oxo-1-azabicyclo [3.2.0] hept-2-ene-2-carboxylic acid p-nitrobenzyl ester sulfoxide, 6-ethyl-3-pantetheinyl-7-oxo-1-azabicyclo [3.2.0] hept-2-ene-2-carboxylic acid o,p-dinitrobenzyl ester sulfoxide, (5,6-trans)-6-(1-hydroxyethyl)-3-isopropylidenpantetheinyl-7-oxo-1-azabicyclo [3.2.0] hept-2-ene-2-carboxylic acid p-nitrobenzyl ester sulfoxide, (5,6-cis)-6-(1-hydroxyethyl)-3-isopropylidenepantetheinyl-7-oxo-1-azabicyclo [3.2.0]

hept-2-ene-2-carboxylic acid p-nitrobenzyl ester sulfoxide, (5,6-trans)-6-(1-hydroxyethyl)-3-isopropylidene-pantetheinyl-7-oxo-1-azabicyclo [3.2.0] hept-2-ene-2-carboxylic acid p-bromobenzyl ester sulfoxide, (5,6-trans)-6-(1-hydroxyethyl)-3-isopropylidenepantetheinyl -7-oxo-1-azabicyclo [3.2.0] hept-2-ene-2-carboxylic acid o,p-dinitrobenzyl ester sulfoxide, (5,6-cis)-6-(1-hydroxyethyl)-3-isopropylidenepantetheinyl-7-oxo-1-azabicyclo [3.2.0] hept-2-ene-2-carboxylic acid p-methoxybenzyl ester sulfoxide, 6-ethyl-3-isopropylidene-pantetheinyl-7-oxo-1-azabicyclo [3.2.0] hept-2-ene-2-carboxylic acid p-nitrobenzyl ester sulfoxide, 6-(1-hydroxyethyl)-3-isopropylidenepantetheinyl-7-oxo-1-azabicyclo [3.2.0] hept-2-ene-2-carboxylic acid p-nitrobenzyl ester sulfoxide, 3-cyclohexylidenepantetheinyl-6-ethyl-7-oxo-1-azabicylco [3.2.0] hept-2-ene-2-carboxylic acid p-nitrobenzyl ester sulfoxide, 3-cyclo-hexylidenepatetheinyl-6-(1-hydroxyethyl)-7-oxo-1-azabicyclo [3.2.0] hept-2-ene-2-carboxylic acid p-nitrobenzyl ester sulfoxide, 6-ethyl-3-methylidene-pantetheinyl-7-oxo-1-azabicyclo [3.2.0] hept-2-ene-2-carboxylic acid benzyl ester sulfoxide, 6-(1-hydroxyethyl)-3-methylidenepantetheinyl

-7-oxo-1-azabicyclo [3.2.0] hept-2-ene-2-carboxylic acid p-nitrobenzyl ester sulfoxide,3-ehylidenepantetheinyl -6-(1-hydroxyethyl)-7-oxo-1-azabicyclo [3.2.0] hept-2-ene-2-carboxylic acid o,p-dinitrobenzyl ester sulfoxide,3-benzylidenepantetheinyl-6-ethyl-7-oxo-1-azabicyclo [3.2.0] hept-2-ene-2-carboxylic acid o,p-dinitrobenzyl ester sulfoxide, 3-benzylidenepantetheinyl-6-(1-hydroxyethyl)-7-oxo-1-azabicyclo [3.2.0] hept-2-ene-2-carboxylic acid o,p-dinitrobenzyl ester sulfoxide, 6-(1-hydroxyethyl)-3-(1-methylbenzylidene)pantetheinyl-7-oxo -1-azabicyclo [3.2.0] hept-2-ene-2-carboxylic acid p-nitrobenzyl ester sulfoxide, 3-dibenzylidenepantetheinyl-6-(1-hydroxyethyl)-7-oxo-1-azabicyclo [3.2.0] hept-2-ene-2-carboxylic acid benzyl ester sulfoxide, 6-ethyl-3-ethylidene-pantetheinyl-7-oxo-1-azabicyclo [3.2.0] hept-2-ene-2-carboxylic acid p-nitrobenzyl ester sulfoxide, 3-ethylidene-pantetheinyl-6-(1-hydroxyethyl)-7-oxo-1-azabicyclo [3.2.0] hept-2-ene-2-carboxylic acid p-nitrobenzyl ester sulfoxide are mentioned.

The compounds of the present invention may be produced according to EUROPEAN PATENT APPLICATION Publication Number 48999 by the following process:

(A) Antibiotic OA-6129A, OA-9129$B_1$ or OA-6129$B_2$, or salt thereof having formula 1-a obtained by cultivating Streptomyces sp. OA-6129 (deposited in Fermentation Research Institute, Japan, on September 26,1980, which was accepted as the international deposit FERM BP-11 on May 1,1981, according to BUDAPEST TREATY ON THE INTERNATIONAL RECOGNITION OF THE DEPOSIT OF MICROORGANISMS FOR THE PURPOSE OF PATENT PROCEDURE) in a nutrient culture medium, is esterified to obtain an ester thereof by the method known per se.

The antibiotic OA-6129A, OA-6129$B_1$ or OA-6129$B_2$, or salt thereof may be treated with substituted or unsubstituted benzyl halide, for example, such as benzyl chloride, benzyl bromide, p-nitrobenzyl chloride, p-nitrobenzyl bromide, p-methcxybenzyl bromide, 2,4-dinitrobenzyl chloride and p-bromobenzyl bromide, to form ester thereof. Generally, the reaction is preferably carried out in ar inert liquid medium. Useful inert liquid media are, for example, halogenated hydrocarbons such as chloroform and methylene chloride; amides such as dimethyl formamide and hexamethylphosphoramide;

ethers such as tetrahydrofuran and dioxane; esters such as ethyl acetate and n-butyl acetate; and ketones such as acetone and methylethyl ketone. These liquid media may be used alone or as a mixture of more than two of them if required.

The reaction temperature is not critical but may be widely varied depending on the kind of halide and liquid media employed. It may be selected in the range that antibiotic OA-6129A, OA-6129$B_1$ or OA-6129$B_2$ is not much decomposed. Generally a reaction temperature below 60°C, preferably in the range between zero and 40°C, and more preferably between 5°C and room temperature is advantageous. Accelerator for the reaction such as trimethylamine, triethylamine, pyridine and dicyclohexyl carbodiimide may be added to said reaction if required.

The reaction can be completed within 1 to 24 hours, usually within 3 to 12 hours under said conditions.

Antibiotic OA-6129A, OA-6129$B_1$ or OA-6129$B_2$ to be allowed to react with said halide is not necessarily used after isolation but may be used in the form of a broth of antibiotic OA-6129A-producing microorganism mentioned above, in the form of

the cultured broth after removal of cells, or in the form of a crude preparation of the antibiotic that is partially purified according to the isolation and purification process mentioned above. As such a partially-purified preparation, there are mentioned, for example, a concentrate of eluate from active carbon on which the cultured broth is adsorbed; a concentrate of eluate from DIAION HP-20 (Mitsubishi Chemical Industries Ltd.) on which the cultured broth is adsorbed; a concentrate obtained by adsorbing the concentrate mentioned above on QAE-Sephadex (Pharmacia Fine Chemicals AB), eluting with a sodium chloride-gradient in phosphate buffer solution and desalting with active carbon; and a concentrate of butanol extract at pH 3.5 and low temperature.

Esters of antibiotic OA-6129A, OA-6129$B_1$ or OA-6129$B_2$ thus obtained may be isolated and purified from the reaction mixture by various methods known per se. For example, at first, the reaction mixture is poured into aqueous medium in order to remove water-soluble impurities like side-products. At that time, a neutral buffer solution is desirably used as the aqueous medium to maintain neutral pH of the mixture. Then,

the mixture is treated with practically water-immiscible non-polar organic solvent and the ester of antibiotic OA-6129A, OA-6129$B_1$ or OA-6129$B_2$ is extracted into the organic solvent layer. Salts such as sodium chloride and ammonium sulfate may be added to the extraction mixture to obtain higher extraction efficiency by the salting-out effect.. The organic solvent layer is dried with Glauber's salt. The ester may be isolated and purified according to the methods known per se, for example, by the appropriate combination of gel filtration with BIOBEADS S-X3 (BioRads Co.) or Sephadex LH-20 (Pharmacia Co.) and adsorption chromatography with silica gel, alumina or FLORIGIL (Florigin Co.), or by repeated use thereof if required.

(B) Substituted or unsubstituted benzyl esters of antibiotic OA-6129A, OA-6129$B_1$ or OA-6129$B_2$ obtained can be converted to cyclic ketal or cyclic acetal derivatives in which $\alpha$- and $\gamma$-hydroxyl groups bound to the carbon atom at 3-position of the pantoyl group are etherified simultaneously. The ketal or acetal formation may be accomplished by the method known per se.

For example, esters of antibiotic OA-6129A,

OA-6129$B_1$ or OA-6129$B_2$ can be converted to cyclic ketal or acetal derivatives thereof by allowing the ester to react with ketone or aldehyde derivatives. Generally the reaction is preferably carried out in the reaction agent employed as the reaction solvent. As suitable reagents there are mentioned, for example, acetone, 2,2-dimethoxypropane, formaldehyde, 2,2-dimethoxyethane, acetaldehyde, benzaldehyde, propionaldehyde, acetophenone, diphenylketone, dibenzylketone, 1,1-diphenylketone and cyclohexanone. These reagents may be employed alone or as a mixture of more than two of them if required. Inert solvents may also be mixed depending on the solubility of the ester of antibiotic OA-6129A, OA-6129$B_1$ and OA-6129$B_2$.

The reaction temperature is not critical but may be widely varied depending on the kind of liquid medium used. It may be selected in the range that the ester of antibiotic OA-6129A, OA-6129$B_1$ or OA-6129$B_2$ is not much decomposed. Generally a reaction temperature below 60°C, preferably in the range between -40°C and 40°C, and more preferably between 0°C and room temperature is advantageous.

Accelerator for the reaction such as p-toluenesulfonic acid, boron trifluoride etherate and zinc

chloride may be added to the said reaction if required.

The reaction can be completed within 30 minutes to 12 hours, usually within 2 to 5 hours under said conditions.

Cyclic ketal or cyclic acetal derivatives of said esters thus obtained may be isolated and purified from the reaction mixture by methods known per se. For example, the reaction mixture is treated with amines after completion of reaction to inactivate excess of the accelerator for the reaction, and then poured into practically water-immiscible non-polar organic solvent such as ethyl acetate, benzene and chloroform. The organic phase is washed with aqueous medium to remove water-soluble impurities like side-products. At that time, a neutral buffer solution is desirably used as the aqueous medium. The organic solvent layer is concentrated to dryness. The aimed cyclic ketal or cyclic acetal derivatives may be isolated and purified according to the method known per se, for example, by the appropriate combination of chromatographies with silica gel, BIOBEADS (Bio-Rads Labs.) and Sephadex LH-20 (Pharmacia Fine Chemicals AB), or by repeated use thereof if required.

(C) S-Oxidation of the cyclicketal or cyclic acetal derivatives thus obtained of esters of antibiotic OA-6129A, OA-6129$B_1$ or OA-9129$B_2$ or derivatives thereof obtained by etherifying simultaneously α- and γ-hydroxyl groups at 3-position of the pantoyl group can be performed according to the method known per se, for example, that is often employed in the case of S-oxidation of sulfur-containing β-lactam antibiotics such as penicillin- and cephalosporin-antibiotics. For example, mild oxidizing agents such as perbenzoic acid, phenyl dichloroiodide, hydrogen peroxide, selenium dioxide and sodium periodate may be employed for the reaction, which do not act on the carbapenem nucleus practically. As the mild oxidizing agents, peracid is suitable. Preferably perbenzoic acid and m-chloroperbenzoic acid are mentioned, and most preferably substituted perbenzoic acid such as m-chloroperbenzoic acid is used.

The reaction between esters of antibiotic OA-6129A, OA-6129$B_1$ or OA-6129$B_2$, or derivatives thereof obtained by etherifying simultaneously α- and γ-hydroxyls of the pantoyl group and the oxidizing agents, may be advantageously carried out in inert solvents such as methylene chloride, chloroform and carbon tetrachloride at room temperature or a lower

temperature, preferably under mild conditions such as at a temperature between about -40°C and about 20°C. The reaction can usually be completed within 3 minutes to 3 hours under said conditions. An amount of oxidizing agent used for the oxidation may be widely varied depending on the kind of oxidizing agent and reaction conditions. Generally, a range between 0.8 and 1.8 molar equivalents per mole of the compound to be treated, and preferably between 1.0 and 1.3 molar equivalents is suitale.

After completion of the reaction, the S-oxidation products can be isolated and purified according to various methods known per se, which are usually employed for isolation and purification of carbapenem antibiotics. For example, after the reaction is completed, solids are removed from the reaction mixture by filtration. To the filtrate is added a buffer solution such as phosphate buffer solution in order to keep neutral pH during further processes. The aimed compounds can be isolated and purified by the following procedures alone or by an appropriate combination thereof, for example, combination of adsorption on active carbon, Amberlite XAD-2 (Rhom and Haas Co.) or DIAION HP-20 (Mitsubishi Industries Ltd.) and elution with methanol/water or acetone/water;

adsorption and elution procedures with ion exchangers such as Dowex 1X2 (Dow Chemicals Co.), Dowex 50X2 (Dow Chemicals Co.) and QAE-Sephadex A-25 (Pharmacia Fine Chemicals AB) and Biogel P-2 (Bio-Rads Labs.); column or thin-layer chromatography with cellulose, Avicel SF (American Viscose Corp.), DEAE-cellulose Whatman DE-32 (Whatman Inc.), DEAE-Sephadex A-25 (Pharmacia Fine Chemicals AB), silica gel and alumina; forced precipitation by addition of solvents such as acetone; and lyophilization.

Hydrolysates (carboxylic acids) of esters obtained from the compounds according to the present invention or salts thereof have broad antimicrobial spectrum. On various microorganims, especially on Gram-positive bacteria belonging to , for example, Staphylococcus, Sarcina and Bacillus, extremely high antimicrobial activity is shown. In addition, even on Gram-negative bacteria belonging to, for example, Alcaligenes and Comamonas, they have very high activity. The esters show a similar antimicrobial activity on an agar medium containing 10% horse serum (Nippon Bio-Supply Center). Carboxylic acid forms of the cyclic ketals or cyclic acetals according to the present invention obtained by etherifying simultaneously α-and γ-hydroxyls of pantoyl group being contained in esters of antibiotic OA-6129A, OA-6129$B_1$ or OA-6129$B_2$, demonstrate noticeable antimicrobial activity on Gram-negative bacteria belonging to, for example, <u>Escherichia</u> and <u>Klebsiella</u>.

It is characteristic that the compounds according to the present invention have remarkably high antimicrobial activity on Gram-negative bacteria, for example, such as <u>Citrobacter</u>, <u>Proteus</u>, <u>Enterobacter</u>, <u>Klebsiella</u> and <u>Serratia</u> that are resistant against antibiotics containing β-lactam ring.

In addition, S-oxides of cyclic ketal and cyclic acetal derivatives according to the present invention, in which α- and γ-hydroxyls of pantoyl group being containing in esters of antibiotic OA-6129A, OA-6129$B_1$ or OA-6129$B_2$ are simultaneously etherified, can be converted to useful substituted derivatives that have an ethyl or 1-hydroxyethyl group at 6-position, or sulfur-containing chain at 3-position of 7-oxo-1-azabicyclo [3.2.0] hept-2-ene-2-carboxylic acid nucleus, by the method described in the specification of EUROPEAN PATENT APPLICATION Publication Number 11173 mentioned above. Therefore, they are useful reaction intermediates.

The following examples further illustrate the present invention.

Example 1

A process for producing benzylester of antibiotic OA-6129A

Sodium salt of antibiotic OA-6129A, 44.6mg, was dissolved in 8.0ml of dimethylformamide. To the solution was added 0.25ml of triethylamine, and then 0.18ml of benzyl bromide with stirring in an ice bath. After 30-minute-reaction at said temperature, the reaction was continued for further 3 hours at room temperature.

The reaction mixture was poured into 100ml of ethyl acetate. The organic layer was washed with 20ml of saturated solution of sodium chloride dissolved in 0.01 M phosphate buffer solution (pH 8.4). The aqueous layer was treated with 100ml of methylene chloride for reextraction. The extracts were combined, dried with sodium sulfate (anhydrous) and evaporated in vacuo. The residue was dissolved in a small amount of benzene and adsorbed onto a Biobeads SX-3 column. The eluate with benzene was subjected to silica gel thin-layer chromatography with a mixture of benzene/acetone (1/1) as a developing solvent. The fraction showing UV absorption at Rf 0.39 was eluted and evaporated to dryness in vacuo.

The residue was dissolved in a small amount of methylene chloride and adsorbed onto a column packed with 12g of silica gel in benzene/acetone (2/1). Elution was carried out successively with mixtures of

benzene/acetone (2/1), (1/1) and (1/3), and acetone. The acetone eluate was evaporated in vacuo to obtain 21.4mg of the aimed compound.

The benzyl ester of antibiotic OA-6129A had the following physicochemical properties:

(1) Specific rotation

$[\alpha]_D^{24}$ : 31.5° (c=1.0, $CH_2Cl_2$)

(2) Ultraviolet absorption spectrum

$\lambda_{max}^{CH_2Cl_2}$ nm ($\varepsilon$) : 318 (7400)

(3) Infrared absorption spectrum

$\nu_{max}^{CH_2Cl_2}$ $cm^{-1}$ : 1772 ($\beta$-lactam)

1700 (ester)

1665 (amide)

(4) Nuclear magnetic resonance spectrum

(Internal standard : TMS)

(i) $CD_2Cl_2$ (solvent)

$\delta$ (ppm) :

0.88 (3H, s, $C\underline{H}_3$-$\overset{CH_3}{\overset{|}{C}}$-), 0.97 (3H, s, $CH_3$-$\overset{C\underline{H}_3}{\overset{|}{C}}$-), 1.03 (3H, t, J=7.5Hz, $CH_2$-$C\underline{H}_3$), 1.60-2.10 (3H, m, $C\underline{H}_2$-$CH_3$, OH), 2.39 (2H, t, J=6.5Hz, N-$CH_2$-$C\underline{H}_2$-CO), 2.85-3.67 (12H, m, C-4$H_2$, C-6H, S-$C\underline{H}_2$-$C\underline{H}_2$-N, N-$C\underline{H}_2$-$CH_2$-CO, C-$C\underline{H}_2$-OH, OH or NH), 3.93 (2H, m, C-5H, HO-$\overset{|}{C}\underline{H}$-CO), 4.17 (1H, br, NH or OH), 5.17 (1H, d, J=13.0HZ, C$\underline{H}$H-Ar), 5.32 (1H, d, J=130Hz, CH$\underline{H}$-Ar), 6.73 (1H, br, NH), 7.35 (5H, s, Ar$\underline{H}$)

(ii)$CD_2Cl_2$+ $D_2O$ (solvent)

δ (ppm) :

0.88 (3H, s, $C\underline{H}_3$-$\overset{CH_3}{\overset{|}{C}}$- ), 0.95 (3H, s, $CH_3$-$\overset{C\underline{H}_3}{\overset{|}{C}}$- ), 1.02 (3H, t, J=7.5Hz, $CH_2$-$C\underline{H}_3$ ), 1.55-2.00 (2H, m, $C\underline{H}_2$-$CH_3$ ), 2.39 (2H, t, J=6.5Hz, N-$CH_2$-$C\underline{H}_2$-CO), 2.80-3.67 (11H, m, C-4$H_2$, C-6H, S-$C\underline{H}_2$-$C\underline{H}_2$-N, N-$C\underline{H}_2$-$CH_2$-CO, C-$C\underline{H}_2$-OH), 3.93 (1H, dt, J=3.0Hz, J=9.0Hz, C-5H), 3.93 (1H, s, HO-$\overset{|}{C}\underline{H}$-CO), 5.13 (1H, d, J=13.0Hz, $C\underline{H}$H-Ar), 5.28 (1H, d, J=13.0Hz, CH$\underline{H}$-Ar), 7.35 (5H, s, ArH)

(5) Mass spectrum

MS (m/z) : 455 ($M^+$ - 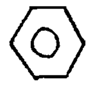 $-CH_3$), 418,

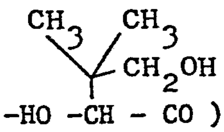

416 ($M^+$ -HO -CH - CO ),

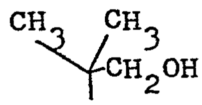

400 ($M^+$ -HO -CH-$CONH_2$ ),

329,

309 (400-$CH_2$ - 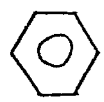 ),

259 ( 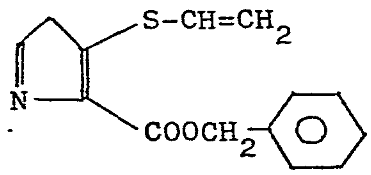 )

Presence of cysteamine and β-alanine was confirmed in the hydrolysate (6N hydrochloric acid, 115°C, 16 hours) of the ester obtained in this process.

Example 2

A process for producing p-nitrobenzyl ester of antibiotic OA-6129A

Antibiotic OA-6129A, 63.5mg, was dissolved in 9.0ml of dimethylformamide. To the solution was added 0.2ml of triethylamine, and then 285 mg of p-nitrobenzyl bromide dissolved in 1.5ml of dimethylformamide, with stirring in an ice bath. After 30-minute-reaction at said temperature, the reaction was continued for further 3 hours at room temperature.

The reaction mixture was poured into 100ml of ethyl acetate and washed with 20ml of saturated solution of sodium chloride dissolved in 0.01M phosphate buffer solution (pH 8.4). The aqueous layer was treated with 100ml of methylene chloride for reextraction. The extracts were combined, dried with sodium sulfate(anhydrous) and evaporated in vacuo. The residue was dissolved in a small amount of methylene chloride and adsorbed onto a column packed with 12g of silica gel in benzene/acetone (1/1). Elution was carried out successively with mixtures of benzen/acetone (1/1) and (1/3), and acetone. From the acetone eluate, the fraction that showed UV absorption at Rf 0.33 in silica gel thin-layer chromatography developed with a mixture of benzen/acetone (1/1), was recovered and evaporated to dryness in vacuo to obtain 36.3mg of the aimed compound.

The p-nitrobenzyl ester of antibiotic OA-6129A

had the following physicochemical properties:

(1) Specific rotation

$[\alpha]_D^{24}$ : 37.5° (c=1.0, $CH_2Cl_2$)

(2) Ultraviolet absorption spectrum

$\lambda_{max}^{CH_2Cl_2}$ nm ($\varepsilon$) : 319 (8400)

270 (10500)

(3) Infrared absorption spectrum

$\nu_{max}^{CH_2Cl_2}$ $cm^{-1}$ : 1770 ($\beta$-lactam)

1700 (ester)

1665 (amide)

(4) Nuclear magnetic resonance spectrum

(Internal standard : TMS)

$CD_2Cl_2$ (solvent)

$\delta$ (ppm) :

0.87 (3H, s, $C\underline{H}_3-\overset{\overset{CH_3}{|}}{C}-$), 0.95 (3H, s, $CH_3-\overset{\overset{C\underline{H}_3}{|}}{C}-$), 1.04 (3H, t, J=7.5Hz, $CH_2-C\underline{H}_3$), 1.5-2.2 (3H, m, $C\underline{H}_2-CH_3$, OH), 2.40 (2H, t, J=6.5Hz, $N-CH_2-C\underline{H}_2-CO$), 2.8-3.7 (12H, m, $C-4H_2$, C-6H, $S-C\underline{H}_2-C\underline{H}_2-N$, $N-C\underline{H}_2-CH_2-CO$, $C-C\underline{H}_2-OH$, OH or NH), 3.94 (2H, m, C-5H, $HO-\overset{|}{C\underline{H}}-CO$), 4.17 (1H, br, NH or OH), 5.19 (1H, d, J=14Hz, $C\underline{H}H$-Ar), 5.45 (1H, d, J=14Hz, $CH\underline{H}$-Ar), 6.74 (1H, br, NH), 7.63 (2H, d, J=9Hz, ArH), 8.18 (2H, d, J=9Hz, ArH)

Example 3

A process for forming isopropylidene of p-nitrobenzyl ester of antibiotic OA-6129A

p-Nitrobenzyl ester of antibiotic OA-6129A, 110mg, was dissolved in 10ml of acetone. To the solution was added 0.5ml of 2,2-dimethoxypropane, 20mg of anhydrous sodium sulfate, and then 40mg of anhydrous p-toluenesulfonic acid with stirring at room temperature. The reaction was continued for further 3 hours at said temperature.

To the reaction mixture was added 0.1ml of triethylamine dropwise. After stirring for 5 minutes, the mixture was poured into 50ml of ethyl acetate and washed with 20ml of 0.1M phosphate buffer solution (pH 8.4) and then with 20ml of the same buffer solution (pH 6.8). The extract was dried with anhydrous sodium sulfate and evaporated in vacuo. The residue was dissolved in a small amount of methylene chloride and adsorbed onto a column packed with 5g of silica gel in benzene/acetone (5/1). Elution was carried out successively with mixtures of benzene/acetone (5/1), (3/1), (1/1), (2/1) and (1/5). Eluate with benzene/acetone (1/1) was concentrated to obtain 72.0mg of the aimed compound that showed ultraviolet absorption at Rf 0.56 in silica gel thin-layer chromatography developed

with a mixture of bezene/acetone (1/1).

The compound had the following physicochemical properties:

(1) Specific rotation

$[\alpha]_D^{24}$ : 34.9° (c=1.0, $CHCl_3$)

(2) Ultraviolet absorption spectrum

$\lambda_{max}^{CHCl_3}$ nm ($\varepsilon$) : 319 (6200)
270 (9800)

(3) Infrared absorption spectrum

$\nu_{max}^{CHCl_3}$ $cm^{-1}$: 1770 ($\beta$-lactam)
1660 (amide)

(4) Nuclear magnetic resonance spectrum

($CDCl_3$ : solvent; Internal standard : TMS)

$\delta$ (ppm) :

0.97 (3H, s, 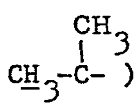 ), 1.03 (3H, s, 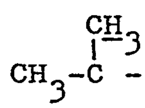 ), 1.07 (3H, t, J=7.5Hz, $CH_2$-$C\underline{H}_3$), 1.40 (3H, s, 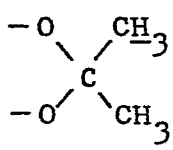 ), 1.43 (3H, s, 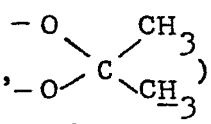 ), 1.7-2.0 (2H, m, -$C\underline{H}_2$-$CH_3$), 2.43 (2H, t, J=6.5Hz, N-$CH_2$-$C\underline{H}_2$-CO), 2.5-3.8 (11H, $C_4$-$\underline{H}_2$, $C_6$-$\underline{H}$, -S-$C\underline{H}_2$-$CH_2$-N, N-$C\underline{H}_2$-$C\underline{H}_2$-CO, C-$C\underline{H}_2$-O-), 3.97 (1H, dt, J=3Hz, 9Hz, $C_5$-$\underline{H}$), 4.03 (1H, s, COC$\underline{H}$-O-), 5.20 (1H, d, J=14Hz, C$\underline{H}$H-Ar), 5.49 (1H, d, J=14Hz, CH$\underline{H}$-Ar), 6.52 (1H, br, N$\underline{H}$), 6.93 (1H, br, N$\underline{H}$), 7.58 (2H, d, J=9Hz, ArH), 8.15 (2H, d, J=9Hz, ArH)

Example 4

A process for producing p-nitrobenzyl ester of antibiotic OA-6129$B_2$

Sodium salt of antibiotic OA-6129$B_2$, 190mg, was dissolved in 6.0ml of dimethylformamide. To the solution was added 0.2ml of triethylamine, and then 210mg of p-nitrobenzyl bromide dissolved in dimethylformamide with stirring in an ice bath. After 5-minute-reaction at said temperature, the reaction was continued for further 3 hours at room temperature. The reaction mixture was poured into 100ml of methylene chloride and washed with 20ml of 0.1M phosphate buffer solution (pH 6.8) twice. The aqueous layer was treated with 100ml of methylene chloride twice for reextraction. The extracts were combined, dried with anhydrous sodium sulfate, and then evaporated in vacuo.

The residue was dissolved in a small amount of methylene chloride and adsorbed onto a column packed with 6g of silica gel in benzene/acetone (1/1). Elution was carried out successively with mixtures of benzene/acetone (1/1), (1/2), (1/3) and (1/5), and acetone. From the fractions eluted with benzene/acetone (1/5) and acetone, 85mg of the aimed compound was obtained that showed ultraviolet absorption at

Rf 0.15 in silica gel thin-layer chromatography developed with a mixture of benzene/acetone (1/4).

The compound had the following physicochemical properties:

(1) Specific rotation

$[\alpha]_D^{24}$ : 41.4° (c=1.0, dioxane)

(2) Ultraviolet absorption spectrum

$\lambda_{max}^{CH_2Cl_2}$ nm ($\varepsilon$) : 320 (10500)

271 (10500)

(3) Infrared absorption spectrum

$\nu_{max}^{CH_2Cl_2}$ $cm^{-1}$ : 1760 ($\beta$-lactam)

1695 (ester)

1640 (amide)

(4) Nuclear magnetic resonance spectrum

(Pyridine-$d_5$)

Only 1 - 5 ppm is shown.

$\delta$ (ppm) :

1.30 (6H, s, $C\underline{H}_3$-C-$C\underline{H}_3$), 1.55 (3H, d, J=7.0Hz, $C\underline{H}_3$-CH), 2.70 (2H, t, J=6.5Hz, NH-$CH_2C\underline{H}_2$CO), 2.90-4.05 (11H, m, C-$4H_2$,C-6H, S-$C\underline{H}_2$-$C\underline{H}_2$NH, NH-$C\underline{H}_2$-$CH_2$-CO, O-$CH_2$-$\overset{|}{\underset{|}{C}}$-), 4.10-4.50 (2H, m, C-5H, C-8H), 4.52 (1H, s, HO-$\overset{|}{C}$H-CO)

(5) Mass spectrum (FD)

m/z : 523 [(M+1) -$CH_3$-$\overset{OH}{\overset{|}{C}H}$-CH=C=O ]

Example 5

A process for forming isopropylidene of p-nitrobenzyl ester of antibiotic OA-6129$B_2$

p-Nitrobenzyl ester of antibiotic OA-6129$B_2$, 20mg, was dissolved in a mixture of acetone, 5.0ml, 2,2-dimethoxypropane, 2.0ml and anhydrous sodium sulfate, 100mg. To the mixture was added 0.5mg of p-toluenesulfonic acid with stirring at room temperature. After 30-minute-reaction, 6$\mu$l of triethylamine was added to the reaction mixture, which was stirred for 5 minutes. The reaction mixture was evaporated in vacuo and 30 ml of methylene chloride was added to the residue. The solution was washed with 20ml of 0.1M phosphate buffer solution. The organic layer was dried with anhydrous sodium sulfated and evaporated in vacuo. The residue was dissolved in a small amount of methylene chloride and adsorbed onto a column packed with 2g of silica gel in benzene/acetone (2/1). Elution was carried out successively with mixtures of benzene/acetone (2/1), (1/1) and (1/2). Fractions eluted with benzene/acetone (1/1) and (1/2) were collected and evaporated to obtain 6.6mg of the aimed isopropylidene compound that showed Rf 0.62 in silica gel thin-layer chromatography developed with a mixture of benzene/acetone (1/4).

The compound had the following physicochemical properties:

(1) Specific rotation

$[\alpha]_D^{24}$ : 55.1° (c=0.5, $CH_2Cl_2$)

(2) Ultraviolet absorption spectrum

$\lambda_{max}^{CHCl_3}$ nm ($\varepsilon$) : 319 (9700)

270 (11900)

(3) Infrared absorption spectrum

$\nu_{max}^{CHCl_3}$ $cm^{-1}$ : 1778 ($\beta$-lactam)

1700 (ester)

1668 (amide)

(4) Nuclear magnetic resonance spectrum

CD $Cl_3$ (solvent)

$\delta$ (ppm) :

$CH_3$ $CH_3$

0.95 (3H, s, $C\underline{H}_3$-C- ), 1.02 (3H, s, $C\underline{H}_3$-C- ), 1.37 (3H, d, J=7.0Hz, $C\underline{H}_3$-CH), 1.40 (3H, s, $C\underline{H}_3$-C(O)(O)-$CH_3$), 1.43 (3H, s, $CH_3$-C(O)(O)-$C\underline{H}_3$), 2.41 (2H, t, J=6.5Hz, NH-$CH_2$-$C\underline{H}_2$-CO), 2.75-3.80 (11H, m, C-4$H_2$, C-6H, S-$C\underline{H}_2$-$C\underline{H}_2$-NH, NH-$C\underline{H}_2$-$CH_2$-CO, O-$CH_2$-C), 4.02 (1H, s, O-CH-CO), 4.00-4.30 (2H, m, C-5H, C-8H), 5.16 (1H, d, J=14.5Hz, C$\underline{H}$H-Ar), 5.44 (1H, d, J=14.5Hz, CH$\underline{H}$-Ar), 6.56 (1H, br, NH), 6.92 (1H, br, NH), 7.55 (2H, d, J=8.0Hz, Ar·$\underline{H}$), 8.12 (2H, d, J=8.0Hz, Ar·$\underline{H}$)

(5) Mass spectrum (FD)

OH

(m/z) : 563 [(M+1)-$CH_3$-CH-CH=C=O]

OH

562 (M-$CH_3$-CH-CH=C=O)

Example 6

A process for producing p-nitrobenzyl ester S-oxide of antibiotic OA-6129A

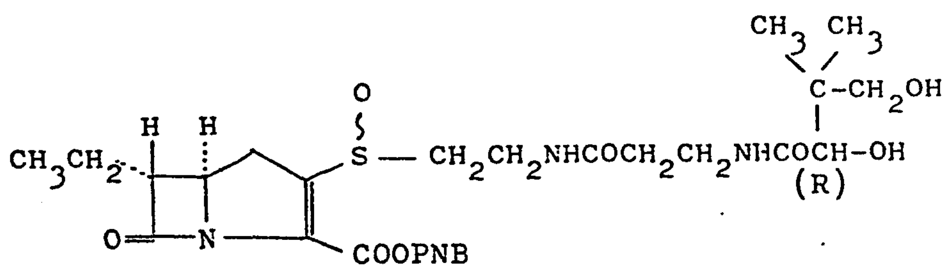

p-Nitrobenzyl ester of antibiotic OA-6129A, 26mg (0.044mmol), was dissolved in 5ml of methylene chloride. To the solution was added 11mg(0.066mmol) of m-chloroperbenzoic acid dissolved in methylene chloride dropwise at -35°C. After 30-minute-reaction at said temperature, the reaction mixture was poured in 100ml of methylene chloride and washed with saturated aqueous solution of sodium bicarbonate. The organic layer was adsorbed onto a column packed with 10ml of silica gel in benzene/acetone (1/1). Elution was carried

out successively with mixtures of benzene/acetone (1/3) and (1/5). Fractions showing ultraviolet absorption at Rf 0.14 in silica gel thin-layer chromatography developed with acetone were collected and evaporated to dryness in vacuo to obtain 16.8mg of the aimed compound.

The physicochemical properties of the compound obtained by treating, with peracid in a similar way, (5R,6R)-6-ethyl-3-(R)-pantetheinyl-7-oxo-1-azabicyclo [3.2.0] hept-2-ene-2-carboxylic acid p-nitrobenzyl ester prepared by treating PS-5 p-nitrobenzyl ester S-oxide with D-pantetheine, accorded with those of the above compound.

The properties are as follows:

(1) Specific rotation

$[\alpha]_D^{24}$ : 11.0° (c=1.0, $CH_2Cl_2$)

(2) Ultraviolet absorption spectrum

$\lambda_{max}^{CH_2Cl_2}$ nm ($\varepsilon$) : 310 sh (7500)

270 (12400)

(3) Infrared absorption spectrum

$\nu_{max}^{CH_2Cl_2}$ cm : 1790 ($\beta$-lactam)

1715 (ester)

1670 (amide)

(4) Nuclear magnetic resonance spectrum

$CD_2Cl_2$(solvent)

$\delta$ (ppm) :

0.88 (3H, s, $\underline{CH_3}$-C($CH_3$)- ), 0.98 (3H, s, $CH_3$-C($\underline{CH_3}$) - ), 1.05 (3H, t, J=7.0Hz, $CH_2$-$\underline{CH_3}$), 1.50-2.00 (3H, m, $\underline{CH_2}$-$CH_3$, OH), 2.39 (2H, t, J=6.0Hz, CO-$\underline{CH_2}$-$CH_2$-NH), 2.95-4.30 (14H, m, C-4$\underline{H}_2$, C-5$\underline{H}$, C-6$\underline{H}$, S-$\underline{CH_2}$-$\underline{CH_2}$-NH, CO-$CH_2$-$\underline{CH_2}$-NH, C-$\underline{CH_2}$-O, O-$\underline{CH}$-CO, OH), 5.10-5.60 (2H, m, $\underline{CH_2}$-Ar), 6.70 (1H, m, NH), 7.30 (1H, m, NH), 7.62 (2H, m, Ar.$\underline{H}$), 8.20 (2H, m, Ar.$\underline{H}$)

(5) Mass spectrm (FD)

(m/z) : 631 (M+Na), 609 (M+1)

Example 7

A process for producing p-nitrobenzyl ester S-oxide of isoprpylidene of antibiotic OA-6129$B_2$

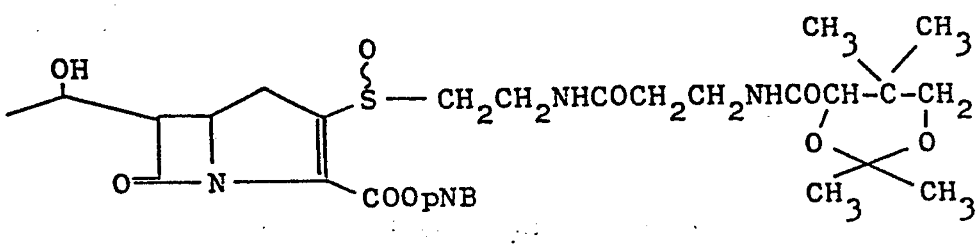

O=Isopropylidene of p-nitrobenzyl ester of antibiotic OA-6129$B_2$, 110mg(0.170mmol), was dissolved in 7.7ml of methylene chloride. To the solution was added 42.9mg(0.187mmol) of m-chloroperbenzoic acid dissolved in methylene chloride dropwise at -30°C, After 30-minute-reaction at said temperature, 0.027ml (0.193mmol) of triethylamine dissolved in methylene chloride was added dropwise at said temperature to

the reaction mixture, which was poured in 20ml of methylene chloride and washed with saturated aqueous solution of sodium bicarbonate. The washed reaction mixture was washed again with phosphate buffer solution (pH 6.80). The organic layer was dried with anhydrous sodium sulfate and evaporated in vacuo. The residue was adsorbed onto a column packed with 5g of silica gel in benzene/acetone (2/1). Elution was carried out successively with mixtures of benzene/acetone (1/1), (1/3) and (1/10). Fractions showing ultraviolet absorption (wave length:2537Å) at Rf 0.24 in silica gel thin-layer chromatography developed with benzene/acetone (1/3), were collected and evaporated to dryness in vacuo to obtain 81.8mg of the aimed compound with a recovery of 72.6%.

The compound had the following physicochemical properties:

(1) Specific rotation

$$[\alpha]_D^{23.5} :+18.5^\circ \ (c=1.0, \ CH\,Cl_3)$$

(2) Ultraviolet absorption spectrum

$$\lambda_{max}^{CHCl_3} \ nm\ (\varepsilon) : 314\ (7400)$$

$$268\ (12100)$$

(3) Infrared absorption spectrum

$$\nu_{max}^{CHCl_3} \ cm^{-1} : 1780\ (\beta\text{-lactam})$$

$$1705\ (\text{ester})$$

1660 (amide)

(4) Nuclear magnetic resonance spectrum

CD $Cl_3$(solvent)

$\delta$ (ppm) :

0.97 (3H, s, $CH_3-\overset{CH_3}{\overset{|}{C}}-$ ), 1.04 (3H, s, $CH_3-\overset{CH_3}{\overset{|}{C}}-$ ), 1.37 (3H, d, J=7.0Hz, $CH_3$-CH-), 1.43 (6H, m, $\overset{O\ \ O}{CH_3-CH_3}$), 2.41 (2H, t, J=6.5Hz, CO-$CH_2$-$CH_2$-NH), 2.80-3.80 (11H, m, S-$CH_2$-$CH_2$-NH, CO-$CH_2$-$CH_2$-NH, C-$4H_2$, C-6H, C-$CH_2$-O-), 4.03 (1H, s, -OCH-CO), 4.00-4.50 (2H, m, C-5H, C-8H), 5.19 (1H, d, J=14.5Hz, $-\underset{\underset{H}{|}}{CH}-Ar$), 5.46 (1H, d, J=14.5Hz, $-\underset{\underset{H}{|}}{CH}-Ar$), 6.17 (1H, br, NH), 6.95 (1H, br, NH), 7.67 (2H, m, Ar.H), 8.17 (2H, d, Ar.H)

(5) Mass spectrm (FD)

(m/z) : 687 (M+Na), 665 (M+1)

Example

A process for producing p-nitrobenzyl 6-(1-hydroxyethyl) -7-oxo-3-phenylthio-1-azabicyclo [3.2.0] hept-2-ene-2-carboxylate

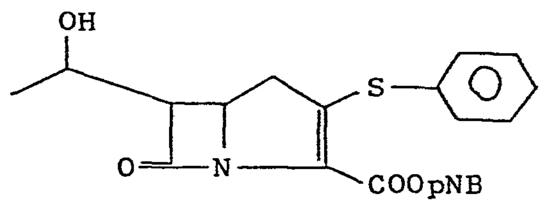

p-Nitrobenzyl ester S-oxide of isopropylidene of antibiotic OA-6129$B_2$, 44mg(0.066mmol), was dissolved in 8ml of dimethylformamide. To the solution was added 28μl(0.198mmol) of triethylamine at -50°C. After addition of 20μl(0.198mmol) of thiophenol, the reaction was continued for further 30 minutes at said temperature. The reaction mixture was poured in 100ml of methylene chloride and washed with phosphate buffer solution (pH 6.8) three times. After dried with anhydrous sodium sulfate, the reaction mixture was evaporated in vacuo and adsorbed onto a column packed with 10ml of silica gel. After development with benzene, elution was carried out with a mixture of benzene/acetone (10/1). Eluates showing ultraviolet

0085407

absorption at Rf 0.86 in silica gel thin-layer chromatography developed with a mixture of benzene/ acetone (3/1), were collected and evaporated in vacuo to obtain 15mg of the aimed compound.

The compound had the following physicochemical properties:

Formula $C_{22}H_{20}N_2O_6S$ (MW=440)

(1) Specific rotation

$[\alpha]_D^{24}$ : 8.9° (c=1.0, CH $Cl_3$)

(2) Ultraviolet absorption spectrum

$\lambda_{max}^{CHCl_3}$ nm (ε) : 321 (13300)

270 (12800)

(3) Infrared absorption spectrum

$\nu_{max}^{CHCl_3}$ $cm^{-1}$ : 1772 (β-lactam)

1700 (ester)

1660 (amide)

(4) Nuclear magnetic resonance spectrum

(Internal standard : TMS)

CD $Cl_3$(solvent)

δ (ppm) :

1.30 (3H, d, J=6.5Hz, CH -C<u>H</u>$_3$), 1.94 (1H, br, OH), 2.65 (2H, d, J=9.0Hz, C-4$H_2$), 3.17 (1H, dd, J=3.0Hz, J=5.5Hz, C-6H), 3.80-4.20 (2H, m, C-5H, C<u>H</u>-$CH_3$), 5.14 (1H, d, J=14.0Hz, C<u>H</u>H-Ar), 5.43 (1H, d, J=14.0Hz, CH<u>H</u>-Ar), 7.20-7.50 (5H, m, S-phe), 7.53

(2H, d, J=9.0Hz, ArH), 8.08 (2H, d, J=9.0Hz, ArH)

(5) Mass spectrm (EI)

(m/z) : 440 (M)

## Claims

What is claimed is:

(1) A compound having the formula:

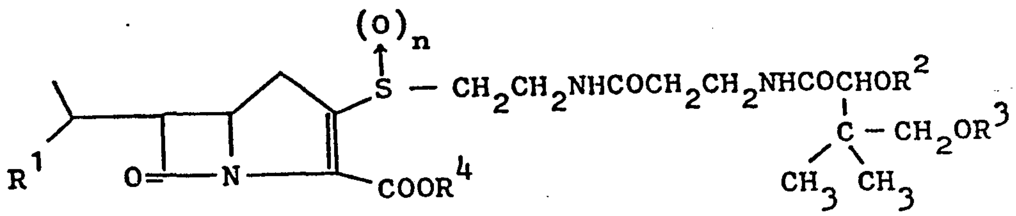

(1)

wherein

n represents zero or 1,

$R^1$ represents a hydrogen atom or a hydroxyl group,

$R^2$ and $R^3$ represent together with each other a group having the formula:

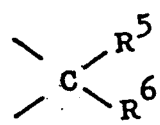

when n is zero,

wherein

$R^5$ and $R^6$ are same or different each other and each represent a hydrogen atom, lower alkyl, phenyl group, a group having a carbon ring of 5 to 6 carbon atoms or diphenylmethyl group, or

$R^2$ and $R^3$ each represent a hydrogen atom or

together with each other a group having the formula:

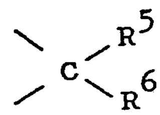

when n is 1,

wherein

$R^5$ and $R^6$ are as mentioned above, and

$R^4$ represents a substituted or unsubstituted benzyl group.

(2) The compound according to claim 1, in which $R^2$ and $R^3$ of formula(1) represent together with each other an isopropylidene group.

(3) The compound according to claim 2, in which $R^1$ of formula(1) represents a hydroxyl group and 5,6-positions form trans- or cis-streostructure.

(4) The compound according to claim 1, in which $R^1$ of formula(1) represents a hydrogen atom and $R^2$ and $R^3$ of formula(1) each represent a hydrogen atom or together with each other an isopropylidene group.

(5) A process for producing compounds of the formula:

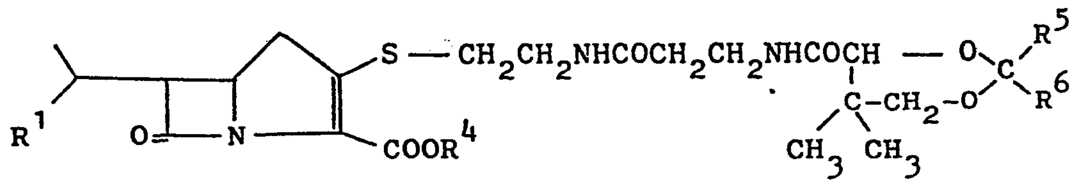

wherein

$R^1$ represents a hydrogen atom or hydroxyl group,

$R^5$ and $R^6$ are same or different each other and represent a hydrogen atom, lower alkyl, phenyl group, a group having a carbon ring of 5 to 6 carbon atoms, benzyl or phenylmethyl group, and

$R^4$ represents a substituted or unsubstituted benzyl group,

which comprises treating a compound of the formula:

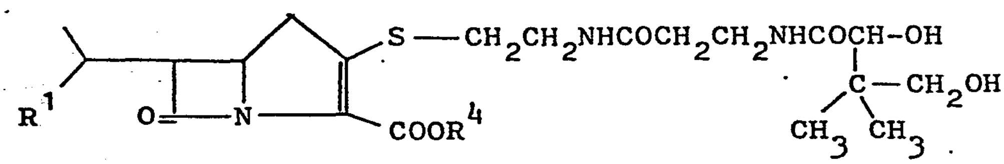

wherein

$R^1$ and $R^4$ are as mentioned above,

with a compound of the formula:

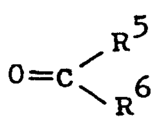

or

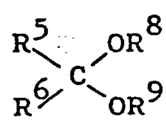

wherein

$R^5$ and $R^6$ are as mentioned above and

$R^8$ and $R^9$ represent a lower alkyl group.

(6) A process for producing compounds of the formula:

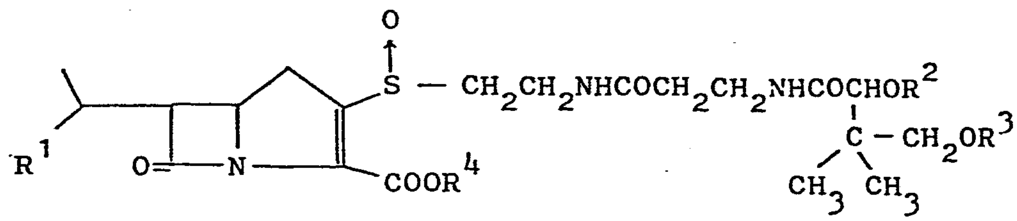

wherein

$R^1$ represents a hydrogen atom or hydroxyl group,

$R^2$ and $R^3$ are same or different each other and represent a hydrogen atom, lower alkyl, a group having a carbon ring of 5 to 6 carbon atoms, benzyl or diphenylmethyl group, and

$R^4$ represents an substituted or unsubstituted benzyl group,

which comprises oxidizing a compound of the formula:

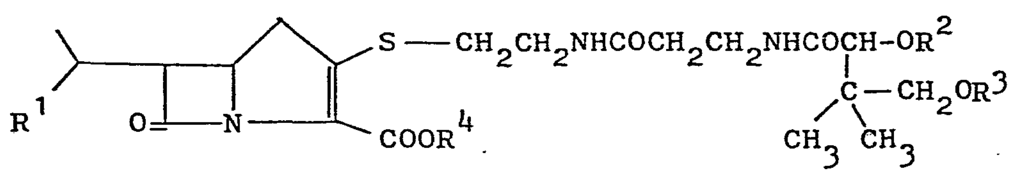

wherein

$R^1$,$R^2$,$R^3$ and $R^4$ are as mentioned above.

European Patent Office

**EUROPEAN SEARCH REPORT**

0085407

Application number

EP 83 10 0772

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 028 497 (BEECHAM) *Claims 1-16* --- | 1 | C 07 D 487/04 // A 61 K 31/40 (C 07 D 487/04 C 07 D 209/00 C 07 D 205/00 ) |
| P | EP-A-0 068 485 (SANRAKU-OCEAN) *Pages 33-37, (Referential examples 3,4* --- | 1,2 | |
| D,P | EP-A-0 048 999 (SANRAKU-OCEAN) *Claims* ----- | 1,3,4 | |
| | | | TECHNICAL FIELDS SEARCHED (Int. Cl. 3) |
| | | | C 07 D 487/00 A 61 K 31/00 |

The present search report has been drawn up for all claims

| Place of search | Date of completion of the search | Examiner |
|---|---|---|
| THE HAGUE | 14-04-1983 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document